# FASCICULE DE BREVET EUROPEEN

(11) **EP 2 931 398 B1**
(45) Date de publication et mention de la délivrance du brevet: **02.06.2021**
(21) Numéro de dépôt: 13815418.2
(22) Date de dépôt: 10.12.2013
(51) Int. Cl.: B01D 15/38, B01D 15/18, C07C 7/12, C07C 7/13, C07C 13/68

(54) **PROCEDE D'ISOLEMENT DE DIAMANDOIDES**
VERFAHREN ZUR ISOLIERUNG VON DIAMANTOIDEN
METHOD FOR ISOLATING DIAMONDOIDS

(30) Priorité: 11.12.2012 FR 1261891
(43) Date de publication de la demande: 21.10.2015
(73) Titulaire: TOTAL SE, 92400 Courbevoie (FR)
(72) Inventeur: SERRES-PIOLE, Coralie, F-64000 Pau (FR); BERRUT, Jean-Bernard, F-64000 Pau (FR)
(74) Mandataire: Hirsch & Associés
(86) Numéro de dépôt international: PCT/EP2013/076100
(87) Numéro de publication internationale: WO 2014/090809

(56) Documents cités:
- US-A- 4 426 292
- US-A- 4 952 749
- LING HUANG ET AL: "A novel method for isolation of diamondoids from crude oils for compound-specific isotope analysis", ORGANIC GEOCHEMISTRY, PERGAMON, AMSTERDAM, NL, vol. 42, no. 5, 25 février 2011 (2011-02-25), pages 566-571, XP028375374, ISSN: 0146-6380, DOI: 10.1016/J.ORGGEOCHEM.2011.02.010 [extrait le 2011-03-04] cité dans la demande

## Description

### Domaine de l'invention

La présente invention concerne un procédé de séparation par chromatographie liquide de diamandoïdes à partir d'un échantillon d'iso-alcanes et de cycloalcanes.

La présente invention concerne également un procédé d'isolement de diamandoïdes à partir d'un mélange de composés organiques, ce procédé mettant en œuvre le procédé de séparation mentionné ci-avant.

La présente invention concerne par ailleurs un kit et un dispositif permettant l'isolement de diamandoïdes par chromatographie liquide à partir d'un mélange de composés organiques.

L'invention concerne également l'utilisation d'un tel kit ou d'un tel dispositif pour séparer des diamandoïdes à partir d'un échantillon d'iso-alcanes et de cycloalcanes ou d'un mélange de composés organiques.

### Arrière-plan technique

Le pétrole est une huile minérale formée d'un mélange de composés organiques piégés dans des formations géologiques très diverses. Ainsi, chaque gisement pétrolier recèle une qualité particulière de pétrole, déterminée par la proportion relative des différents composés organiques le constituant.

Ces composés organiques sont essentiellement des hydrocarbures, parmi lesquels des composés saturés : les n-alcanes, les iso-alcanes et les cycloalcanes, des composés aromatiques, des résines ou encore des asphaltènes.

Parmi les cycloalcanes, on peut citer les diamandoïdes, les terpanes tricycliques, les hopanes, les stéranes. Parmi les iso-alcanes, on peut mentionner le prystane, le phytane.

Les diamandoïdes sont des composés organiques saturés polycycliques à 3 dimensions. Les diamandoïdes se présentent sous forme de cage et peuvent être substitués, ou non, par des groupements alkyles. Parmi ces diamandoïdes, on peut citer, à titre non limitatif, l'adamantane (composé en C₁₀H₁₆), le diamantane (C₁₄H₂₀) ou encore le triamantane (C₁₈H₂₄) ainsi que leurs homologues comprenant au moins une ramification alkyle.

Ces diamandoïdes sont donc des constituants naturels du pétrole, que l'on dénomme également "huile" dans la suite de la présente description. Les diamandoïdes sont couramment rencontrés dans les huiles à des concentrations supérieures à 1 ppm.

En raison de leurs propriétés physico-chimiques uniques (haute stabilité thermique, points de fusion et de pression de vapeur élevés), les diamandoïdes présentent depuis plusieurs années un intérêt grandissant dans un grand nombre de domaines (industrie pharmaceutique, médecine, nanotechnologie, microélectronique,...) dont le domaine pétrolier (G. Ali Mansoori, Advances in Chemical Physics, 136, 207-258, 2007). Il est donc très intéressant d'isoler, de purifier et de concentrer ces diamandoïdes pour une utilisation ultérieure de ces derniers, notamment dans les domaines qui viennent d'être cités.

En outre, du fait de leur grande stabilité dans les huiles, il est intéressant de conduire des analyses, tant qualitatives que quantitatives, sur ces diamandoïdes pour avoir une meilleure compréhension des systèmes pétroliers, en particulier des huiles biodégradées et craquées. De telles analyses permettent notamment d'évaluer la maturité géologique d'un champ d'huile et/ou le niveau de maturité thermique d'huiles, de distinguer deux huiles et/ou de caractériser des mélanges d'huiles, d'évaluer le degré d'avancement de biodégradation des huiles, de déterminer les corrélations huile/huile ou roche-mère/huile. Ceci explique la multiplication des études réalisées visant à isoler et identifier ces diamandoïdes.

De telles analyses qualitatives et/ou quantitatives sont en général conduites par chromatographie gazeuse (GC) ou par chromatographie gazeuse couplée à de la spectrométrie de masse (GC/MS). Les analyses isotopiques sont, quant-à-elles, réalisées par chromatographie gazeuse couplée à la spectrométrie de masse de rapport isotopique (GC/irMS).

Toutefois, au vu de la grande variété des composés formant l'huile et de la quantité très faible de ces composés d'intérêt au sein d'une huile, il s'avère nécessaire de préparer l'échantillon d'huile avant d'effectuer son analyse chromatographique (par GC, GC/MS ou GC/irMS) afin de purifier, d'isoler et/ou de concentrer les composés particuliers que l'on souhaite étudier.

Les procédés décrits dans la littérature pour isoler les diamandoïdes, que ce soit à partir de fractions isolées d'iso-alcanes et de cycloalcanes, ou à partir d'une huile, sont relativement complexes.

On peut en particulier se reporter à la publication scientifique de L. Huang et al. ("A novel method for isolation of diamondoids from crude oils for compound-specific isotope analysis" - Organic Geochemistry - 42 (2011) p.566-571) qui décrit un procédé de séparation de diamandoïdes à partir d'un échantillon d'huile brute. Ce procédé comprend les étapes successives suivantes :
- une première étape de séparation par chromatographie liquide, au travers d'une colonne comprenant un gel de silice activée, pour recueillir une fraction saturée d'hydrocarbures comprenant les n-alcanes et les hydrocarbures cycliques et branchés,
- une étape de concentration sous azote de cette fraction saturée d'hydrocarbures,
- une deuxième étape de séparation de cette fraction saturée d'hydrocarbures concentrée par chromatographie liquide, au travers d'une colonne comprenant un tamis moléculaire (zéolithe de type silicalite ZSM-5), pour recueillir la fraction d'hydrocarbures cycliques et branchés,
- une troisième étape de séparation des diamandoïdes de la fraction d'hydrocarbures cycliques et branchés, cette troisième étape se fondant sur l'inclusion, dans la β-cyclodextrine, des diamandoïdes contenus dans la fraction d'hydrocarbures cycliques et branchés, puis la destruction par hydrolyse acide du polymère de cyclodextrine.

La troisième étape de séparation décrite dans la publication de L. Huang et al. implique la mise en œuvre des manipulations successives suivantes :
- le mélange, sous agitation pendant au moins deux heures, de la fraction d'hydrocarbures cycliques et branchés recueillie après la deuxième étape de séparation, avec de la β-cyclodextrine en solution dans de l'eau déionisée,
- la séparation, par centrifugation, du précipité formé lors du mélange,
- le lavage du précipité par du cyclohexane,
- la mise en solution du précipité lavé dans une solution d'HCl diluée,
- le chauffage (à 80°C pendant au moins 4 heures) de la solution ci-dessus pour obtenir la réaction d'acidolyse de la β-cyclodextrine,
- après refroidissement de la solution, l'extraction des diamandoïdes au moyen de cyclohexane,
- après lavage à l'eau déionisée de la solution extraite puis séchage avec Na₂SO₄, la solution contenant les diamandoïdes a été concentrée sous un flux d'azote.

Cette solution concentrée est ensuite analysée par chromatographie gazeuse couplée à de la spectrométrie de masse (GC/MS).

Si la publication de L. Huang et al. fait état d'une séparation des diamandoïdes, à partir de la fraction d'hydrocarbures cycliques et branchés, qui est réalisée avec succès, il n'en demeure pas moins que la multiplication des manipulations augmente le temps nécessaire pour obtenir les composés recherchés, ce qui se répercute nécessairement sur l'obtention des résultats des analyses ultérieures à conduire sur ces produits.

Cette multiplication des manipulations conduit de surcroît à un très faible rendement de récupération. Ce dernier est typiquement inférieur à 10% de la teneur initiale en diamandoïdes présents dans l'huile brute.

Enfin, la multiplication des manipulations augmente inévitablement les risques de perte et/ou de contamination des échantillons. Par ailleurs, le brevet US 4 952 749 divulgue un procédé de séparation en phase liquide de diamandoïdes d'un mélange d'hydrocarbures, par utilisation de l'effet d'inclusion d'un matériel poreux en particulier une zéolite.

Les cyclodextrines, qui sont obtenues par dégradation enzymatique de l'amidon, sont des oligosaccharides cycliques qui peuvent comprendre de six à huit unités de glucose. On distingue ainsi trois familles de cyclodextrines en fonction du nombre d'unités de glucose formant l'oligosaccharide : ces familles sont désignées α, β et y lorsqu'elles comportent respectivement six, sept et huit unités de glucose.

Les cyclodextrines, et plus particulièrement les β-cyclodextrines, sont connues pour former des complexes d'inclusion dans des solutions aqueuses avec une grande variété de composés polaires et non-polaires, incluant les hydrocarbures aromatiques et les composés comprenant un hétéroatome. La cavité interne de la β-cyclodextrine, qui est essentiellement formée par des atomes de carbone et d'hydrogène, est assez hydrophobe tandis que les faces extérieures sont hydrophiles dans la mesure où tous les groupes hydroxyles des unités glucoses sont tournés vers l'extérieur de la molécule. Le diamètre interne de la cavité hydrophobe de la β-cyclodextrine est compris entre 6,0 et 8,5 Â.

Comme le rapportent les publications de G.Ali Mansoori ("Diamondoid molecules" - Advances in Chemical Physics - 136 (2007) p.207-258) et de C. Leggio et al. ("Study on the structure of host-guest supramolecular polymers" - Macromolecules - 40 (2007) p.5899-5906), l'adamantane, en raison de sa taille, s'insère avec un très bon ajustement dans la cavité de la cyclodextrine et, plus particulièrement, de la β-cyclodextrine.

Le but de la présente invention est de fournir un procédé d'isolement par chromatographie liquide de composés appartenant à la famille des diamandoïdes, que ce soit à partir d'un échantillon de cycloalcanes contenant éventuellement des iso-alcanes, ou plus généralement à partir d'un échantillon d'un mélange de composés organiques, ce procédé palliant au moins partiellement les inconvénients précités.

Plus particulièrement, l'invention se rapporte à un procédé qui permette d'isoler, à partir d'un tel échantillon de cycloalcanes contenant éventuellement des iso-alcanes, ou plus généralement d'un mélange de composés organiques, des diamandoïdes et ce, avec un minimum de manipulations, sans risque de contamination des échantillons et/ou de perte des composés, et dans un temps raisonnable compatible avec la conduite d'analyses qualitatives, quantitatives et/ou isotopiques ultérieures.

### Résumé de l'invention

A cette fin, la présente invention a pour objet un procédé d'isolement de diamandoïdes à partir d'un échantillon d'iso-alcanes et de cycloalcanes comprenant une étape de séparation par chromatographie liquide, cette étape de séparation comprenant :
- l'introduction de l'échantillon dans une colonne comprenant une phase stationnaire comprenant un matériau capable de former des complexes d'inclusion avec les diamandoïdes, le dit matériau étant la β-cyclodextrine,
- l'élution par un éluant choisi parmi les mélanges de n-alcane en C3-C8 et d'alcanol en C1-C4 avec un ratio en volume alcane/alcanol allant de 90/10 à 99,5/0,5 et
- la collecte des fractions éluées.

Dans toute la description qui suit du procédé de l'invention, lorsque l'on indique que l'on part d'un échantillon de cycloalcanes, on prévoit que le procédé est mis en œuvre à partir d'un mélange de cycloalcanes et d'iso-alcanes.

Suivant des modes de réalisation préférés, l'invention comprend une ou plusieurs des caractéristiques suivantes :
- l'éluant est un mélange pentane / isopropanol (98% / 2% vol/vol).
- la colonne est une colonne HPLC reliée à un dispositif HPLC, le procédé étant mis en œuvre avec une pression dans la colonne allant de 10 à 30 bars, avantageusement de 15 à 25 bars.

La présente invention permet de surmonter les inconvénients des procédés de l'art antérieur. Elle fournit plus particulièrement un procédé de séparation d'une fraction de diamandoïdes à partir d'un échantillon de cycloalcanes, à la fois simple et rapide, et comprenant un nombre d'étapes limité par rapport à la troisième étape de séparation du procédé décrit dans la publication scientifique précitée L. Huang et al. Le procédé de séparation selon l'invention permet en particulier d'éviter les risques de contamination et la perte de certains des composés diamandoïdes présents dans l'échantillon de cycloalcanes, risques qui sont inhérents à la multiplication des manipulations de ce procédé de l'art antérieur.

Ce résultat est atteint grâce à la mise en œuvre d'une étape de séparation par chromatographie liquide réalisée au moyen d'une colonne comprenant une phase stationnaire en un matériau capable de former un complexe d'inclusion avec les diamandoïdes, le dit matériau étant la β-cyclodextrine.

Le choix d'une séparation par chromatographie liquide avec une phase stationnaire comprenant un matériau capable de former un complexe d'inclusion avec les diamandoïdes permet d'isoler les diamandoïdes en un nombre très réduit de manipulations par rapport aux procédés de l'art antérieur. Grâce au procédé selon l'invention, la récupération des diamandoïdes à partir d'un échantillon de cycloalcanes est réalisée en un temps bien plus court qu'avec le procédé de l'art antérieur et dans des conditions de mise en œuvre plus fiables, c'est-à-dire avec un risque de contamination et/ou de pertes nettement réduit par rapport à celui inhérent au procédé de l'art antérieur. Enfin, la rapidité et les rendements élevés du procédé de l'invention permettent d'envisager son application à des fins préparatives.

La présente invention concerne également un procédé de séparation d'une fraction de diamandoïdes à partir d'un échantillon d'un mélange de composés organiques ce procédé comprenant les étapes suivantes :
- une première étape de séparation par chromatographie liquide d'une fraction d'iso-alcanes et de cycloalcanes à partir du mélange de composés organiques,
- éventuellement une étape de préparation de la fraction d'iso-alcanes et de cycloalcanes, et
- une deuxième étape de séparation de diamandoïdes à partir d'un échantillon d'iso-alcanes et de cycloalcanes suivant le procédé tel que décrit ci-dessus.

Suivant des modes de réalisation préférés, l'invention comprend une ou plusieurs des caractéristiques suivantes :
- Le procédé comprend :
   - l'introduction du mélange de composés organiques dans une colonne comprenant au moins deux phases stationnaires, la première phase stationnaire étant en un matériau capable d'adsorber les n-alcanes et la deuxième phase stationnaire étant en un matériau capable d'adsorber les composés aromatiques et les composés polaires et ladite deuxième phase stationnaire comprend une silice imprégnée de nitrate d'argent,
   - l'élution par un éluant, et
   - la collecte de la ou des fractions d'iso-alcanes et de cycloalcanes éluées.
- la première phase stationnaire avantageusement, comprend une zéolithe,
- la colonne peut comprendre en outre une troisième phase stationnaire en un matériau capable d'adsorber les composés dont la polarité est supérieure à la polarité des composés adsorbés par le matériau de la deuxième phase stationnaire, et, avantageusement, comprend une silice choisie parmi une silice vierge, une silice greffée par des groupes aminoalkyles, notamment aminopropyles et une silice greffée par des groupes cyanoalkyles et préférentiellement par des groupes cyanopropyles.
- la première étape de séparation est une étape de séparation par chromatographie basse pression, dans laquelle la phase liquide est éluée sous une pression inférieure à 25 bars, avantageusement comprise entre 3 et 10 bars et, de préférence, comprise entre 7 et 9 bars.
- l'éluant est choisi parmi l'iso-octane, le n-octane et leurs mélanges, avantageusement l'iso-octane.
- le procédé comporte une étape intermédiaire de préparation de la fraction d'iso-alcanes et de cycloalcanes, cette préparation comportant une filtration et/ou une concentration par évaporation partielle du solvant.

Les procédés de séparation selon l'invention, qui permettent d'isoler les diamandoïdes à partir d'un échantillon de cycloalcanes ou, de manière plus générale, à partir d'un mélange de composés organiques, présentent également l'avantage d'être facilement reproductibles et de conférer des résultats fiables et ce, dans des délais nettement plus courts que ceux des procédés de l'art antérieur.

L'invention concerne en outre un kit et un dispositif permettant l'isolement de diamandoïdes par chromatographie liquide. Un tel kit comprend :
- une première colonne (1) comprenant au moins deux phases stationnaires, la première phase stationnaire (11) étant en un matériau capable d'adsorber les n- alcanes et la deuxième phase stationnaire (12) étant en un matériau capable d'adsorber les composés aromatiques et les composés polaires et ladite deuxième phase stationnaire comprend une silice imprégnée de nitrate d'argent, éventuellement une troisième phase stationnaire (13) en un matériau capable d'adsorber les composés dont la polarité est supérieure à la polarité des composés adsorbés par le matériau de la deuxième phase stationnaire, et
- une deuxième colonne (2) comprenant une phase stationnaire (16) en un matériau capable de former des complexes d'inclusion avec les diamandoïdes, ledit matériau étant la β-cyclodextrine.

Suivant des modes de réalisation préférés, le kit selon l'invention comprend une ou plusieurs des caractéristiques suivantes :
- le kit comprend un ou plusieurs moyens choisis parmi :
   des moyens (20) permettant de relier la première colonne (1) à des moyens de mise sous pression (22),
   des moyens (34) permettant de relier la seconde colonne (2) à un dispositif HPLC (29),
   des moyens (23, 34) permettant l'introduction des échantillons dans chacune des colonnes (1, 2),
   des moyens (26, 33) de collecte d'échantillon,
   des moyens (25, 32) d'analyse.

L'invention concerne encore un dispositif permettant l'isolement de diamandoïdes à partir d'un échantillon d'un mélange de composés organiques. Un tel dispositif comprend :
- une première colonne (1) comprenant au moins deux phases stationnaires, la première phase stationnaire (11) étant en un matériau capable d'adsorber les n-alcanes et la deuxième phase stationnaire (12) étant en un matériau capable d'adsorber les composés aromatiques et les composés polaires et ladite deuxième phase stationnaire comprend une silice imprégnée de nitrate d'argent, éventuellement une troisième phase stationnaire (13) en un matériau capable d'adsorber les composés dont la polarité est supérieure à la polarité des composés adsorbés par le matériau de la deuxième phase stationnaire, et
- une deuxième colonne (2) comprenant une phase stationnaire (16) en un matériau capable de former des complexes d'inclusion avec les diamandoïdes, ledit matériau étant la β-cyclodextrine,
- des moyens (14, 23) d'introduction de l'échantillon dans la première colonne (1),
- des moyens (22) d'introduction d'un premier éluant dans la première colonne (1),
- éventuellement des moyens (21) de mise sous pression de la première colonne (1),
- éventuellement des moyens (25) de détection et d'analyse des fractions éluées en sortie de première colonne (1),
- des moyens (26) de collecte des fractions éluées en sortie de première colonne (1),
- des moyens (34) d'introduction dans la deuxième colonne, des fractions éluées en sortie de première colonne (1),
- des moyens (28, 30) d'introduction dans la deuxième colonne (2), d'un deuxième éluant,
- des moyens (29) de mise sous pression de la deuxième colonne (2),
- éventuellement des moyens (32) de détection et d'analyse des fractions éluées en sortie de deuxième colonne (2), et
- des moyens de collecte des fractions éluées en sortie de deuxième colonne.
- éventuellement des moyens (35, 36) de contrôle et d'automatisation du procédé, en particulier des moyens permettant le contrôle de l'introduction des échantillons dans les colonnes, et/ou la récupération des fractions éluées en sortie de colonne, et/ou le contrôle des moyens d'analyse, et/ou la récupération et le stockage des données analytiques,

Selon un mode particulier de réalisation, le procédé de séparation de diamandoïdes à partir d'un mélange de composés organiques selon l'invention est mis en œuvre au moyen du kit et/ou au moyen du dispositif conformes à l'invention, en particulier lorsque le mélange de composés organiques est un pétrole brut ou une huile ou un mélange huileux issu du traitement du pétrole, notamment par raffinage.

L'invention se rapporte par ailleurs à une utilisation du kit et/ou du dispositif pour séparer des diamandoïdes à partir d'un mélange de composés organiques, ce mélange pouvant notamment provenir d'un gisement de pétrole brut, d'huiles de schiste ou de coupes de pétrole.

### Brève description des figures

La figure 1 est une représentation schématique d'un mode de réalisation du kit selon l'invention.
La figure 2 est une représentation schématique d'un mode de réalisation du dispositif de séparation selon l'invention, ce dispositif comprenant notamment les pièces du kit de séparation selon l'invention.
La figure 3 est un chromatogramme représentant l'analyse ELSD (Evaporativc Light Scattering Détection ou détection par diffraction de lumière évaporative) de 8 standards purs en solution dans de l'iso-octane et l'analyse par Réfractométrie Différentielle (RD) de l'adamantane en solution dans de l'iso-octane.
La figure 4 est un histogramme représentatif du pourcentage total de collecte des diamandoïdes dans les fractions recueillies en sortie de colonne de β-cyclodextrine après mise en œuvre du procédé selon l'invention.

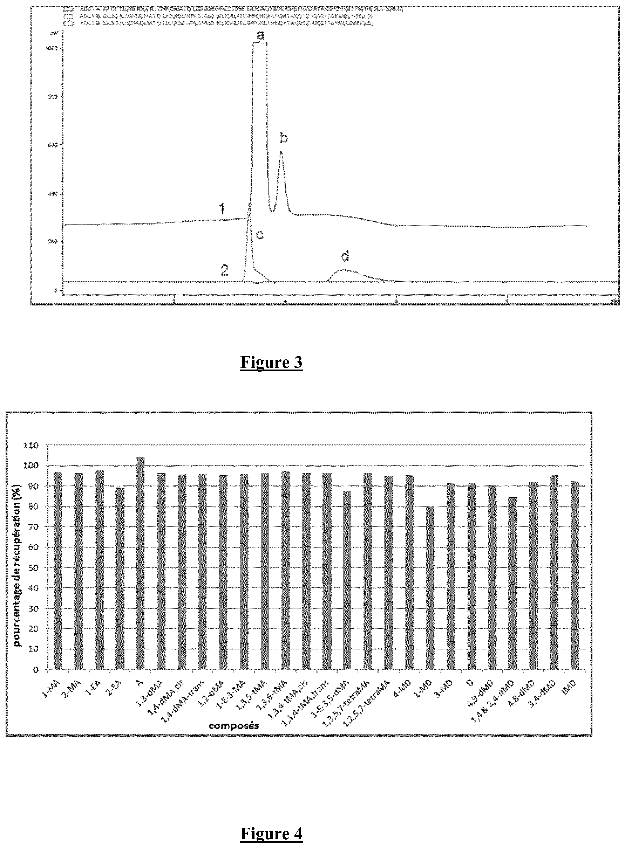

Afin de permettre l'identification des diamandoïdes repérés par leur abréviation couramment utilisée telle qu'elle apparaît sur la figure 4, on pourra se reporter au tableau 1 ci-dessous qui précise, pour chaque diamandoïde, sa formule brute, son numéro d'enregistrement CAS ainsi que son abréviation.

**Tableau 1**

| **Composés** | **Abréviations** | **Formule brute** | **N °** CAS |
|---|---|---|---|
| Adamantane | A | C₁₀H₁₆ | 281-23-2 |
| 1-Me-adamantane | 1-MA | C₁₁H₁₈ | 768-91-2 |
| 1,3-diMe-adamantane | 1,3-dMA | C₁₂H₂₀ | 702-79-4 |
| 1,3,5-triMe-adamantane | 1,3,5-tMA | C₁₃H₂₂ | 707-35-7 |
| 1,3,5,7-tetraMe-adamantane | 1,3,5,7-tetraMA | C₁₄H₂₄ | 1687-36-1 |
| 2-Me-adamantane | 2-MA | C₁₁H₁₈ | 700-56-1 |
| Cis-1,4-diMe-adamantane | 1,4-dMA,cis | C₁₂H₂₀ | 24145-89-9 |
| Trans-1,4-diMe-adamantane | 1,4-dMA,trans | C₁₂H₂₀ | 24145-88-8 |
| 1,3,6-triMe-adamantane | 1,3,6-tMA | C₁₃H₂₂ | 24139-37-5 |
| 1,2-diMe-adamantane | 1,2dMA | C₁₂H₂₀ | 16207-81-1 |
| Cis-1,3,4-triMe-adamantane | 1,3,4-tMA,cis | C₁₃H₂₂ | 24145-90-2 |
| Trans-1,3,4-triMe-adamantane | 1,3,4-tMA,trans | C₁₃H₂₂ | 24145-91-3 |
| 1,2,5,7-tetraMe-adamantane | 1,2,5,7-tetraMA | C₁₄H₂₄ | 34946-70-7 |
| 1-Et-adamantane | 1-EA | C₁₂H₂₀ | 770-69-4 |
| 1-Et-3-Me-adamantane | 1-E-3-MA | C₁₃H₂₂ | 1687-34-9 |
| 1-Et-3,5-diMe-adamantane | 1-E-3,5-dMA | C₁₄H₂₄ | 1687-35-0 |
| 2-Et-adamantane | 2-EA | C₁₂H₂₀ | 14451-87-7 |
| Diamantane | D | C₁₄H₂₀ | 2292-79-7 |
| 4-Me-diamantane | 4-MD | C₁₅H₂₂ | 28375-86-2 |
| 4,9-diMe-diamantane | 4,9-dMD | C₁₆H₂₄ | 70459-27-7 |
| 1-Me-diamantane | 1-MD | C₁₅H₂₂ | 26460-76-4 |
| 1,4 et 2,4-diMe-diamantane | 1,4 & 2,4-dMD | C₁₆H₂₄ | 74340-66-8 74340-67-9 |
| 4,8-diMe-diamantane | 4,8-dMD | C₁₆H₂₄ | 70340-68-0 |
| TriMe-diamantane | tMD | C₁₇H₂₆ | |
| 3 -Me-diamantane | 3-MD | C₁₅H₂₂ | 30545-28-9 |
| 3,4-diMe-diamantane | 3,4-dMD | C₁₆H₂₄ | 70340-69-1 |
| Triamantane | T | C₁₈H₂₄ | 13349-10-5 |
| 9-Me-triamantane | 9-MT | C₁₉H₂₆ | 67615-85-4 |
| DiMe-triamantane | dMT | C₂₁H₃₀ | |

### Description détaillée

L'invention concerne en premier lieu un procédé de séparation de diamandoïdes à partir d'un échantillon de cycloalcanes contenant éventuellement des iso-alcanes, ce procédé comprenant une étape de séparation par chromatographie liquide, cette étape de séparation comprenant :
- l'introduction de l'échantillon dans une colonne comprenant une phase stationnaire comprenant un matériau capable de former des complexes d'inclusion avec les diamandoïdes,
- l'élution par un solvant ou un mélange de solvants, et
- la collecte des fractions éluées.

Les diamandoïdes constituent une famille de composés bien connus de l'homme du métier. Ce sont des composés organiques saturés polycycliques de structure tridimensionnelle, sous forme de cage, éventuellement substitués par des groupements alkyls. Le tableau 1 ci-dessus présente les principaux diamandoïdes connus.

L'échantillon de cycloalcanes utilisé comme matière première du procédé est avantageusement obtenu à partir d'un mélange de composés organiques, en particulier de pétrole brut ou d'une huile ou d'un mélange huileux issu du traitement du pétrole par un procédé connu, notamment par raffinage. Le mélange de composés organiques provient, directement ou après traitement, d'un gisement de pétrole brut, d'un gisement d'huile de schiste, d'une coupe pétrolifère.

Les échantillons de cycloalcanes utilisés comme matière première comprennent généralement aussi des iso-alcanes. Ces mélanges d'iso-alcanes et de cycloalcanes sont en effet préférentiellement obtenus au moyen d'un procédé qui permet leur séparation à partir d'un mélange de composés organiques, tels que les mélanges dérivés du pétrole décrits ci-dessus. Hormis les diamandoïdes, les échantillons de cycloalcanes utilisés comme matière première comprennent généralement les composés suivants : parmi les cycloalcanes, on peut citer les terpanes tricycliques, les hopanes, les stéranes, parmi les iso-alcanes, on peut mentionner le prystane, le phytane.

La colonne de chromatographie est choisie avec des dimensions adaptées à la taille de l'échantillon à traiter.

Le matériau capable de former des complexes d'inclusion avec les diamandoïdes peut être choisi parmi ceux bien connus de l'homme du métier pour présenter cette propriété, notamment, les cyclodextrines. Les complexes d'inclusion résultent d'un ensemble d'interactions incluant : la compatibilité géométrique, les forces de Van der Waals, les interactions électrostatiques et hydrophobes, les liaisons hydrogène. Parmi les familles de composés capables de former des complexes d'inclusion avec des diamandoïdes on peut mentionner la thiourée et la sélénourée (R.Gopal et B. Robertson, Acta, Cryst. (1989), C45 , 257-259). De préférence, le matériau capable de former des complexes d'inclusion avec les diamandoïdes est choisi parmi l'a-cyclodextrine, la β-cyclodextrine, la γ-cyclodextrine. Avantageusement ce matériau est la β-cyclodextrine.

On peut prévoir une étape de préparation du mélange d'iso-alcanes et de cycloalcanes préalable à l'étape de séparation. Cette étape de préparation peut notamment consister en une étape de filtration de l'échantillon d'iso-alcanes et de cycloalcanes et/ou une étape de concentration par évaporation.

L'éluant est choisi parmi les solvants et les mélanges de solvant apolaires. De préférence il est choisi parmi les mélanges de n-alcane et d'alcanol. Avantageusement il est choisi parmi les mélanges de n-alcane en C3-C8 et d'alcanol en C1-C4 avec un ratio en volume alcane/ alcanol allant de 90/10 à 99,5/0,5. Généralement on choisit un mélange de pentane / isopropanol, avantageusement avec un ratio en volume d'environ 98 /2.

Avantageusement l'invention est mise en œuvre à l'aide d'une colonne HPLC reliée à un dispositif HPLC (Chromatographie Liquide à Haute Performance), ce qui permet d'imposer une pression contrôlée dans la colonne de chromatographie. Avantageusement, le procédé est mis en œuvre avec une pression dans la colonne allant de 10 à 30 bars, avantageusement de 15 à 25 bars, de préférence de 17 à 22 bars.

Les fractions éluées sont récupérées en sortie de colonne. Elles peuvent ensuite être analysées. Selon une autre variante, un détecteur est placé en sortie de colonne de chromatographie de façon à analyser les fractions éluées avant leur collecte. Cette dernière variante est préférée car elle permet d'identifier directement les fractions éluées contenant les composés diamandoïdes. Parmi les moyens de détection utilisables, on peut mentionner la détection par diffraction de lumière évaporative ou ELSD, la réfractométrie différentielle notée RD, la spectrométrie de masse notée MS, l'analyse isotopique spécifique de composés.

Grâce au procédé de l'invention, les diamandoïdes sont sélectivement isolés à partir d'un mélange d'iso-alcanes et de cycloalcanes, ces derniers étant élués avant les diamandoïdes, en raison de leur moindre affinité avec la cyclodextrine.

Selon une variante préférée, l'invention concerne un procédé d'isolement de diamandoïdes à partir d'un mélange de composés organiques par un procédé comprenant les étapes suivantes :
- une première étape de séparation par chromatographie liquide d'une fraction d'iso-alcanes et de cycloalcanes à partir du mélange de composés organiques,
- éventuellement une étape de préparation de la fraction d'iso-alcanes et de cycloalcanes, et
- une deuxième étape de séparation de diamandoïdes à partir d'un échantillon d'iso-alcanes et de cycloalcanes suivant le procédé décrit ci-dessus.

Selon cette variante la première étape de séparation d'iso-alcanes et de cycloalcanes à partir du mélange de composés organiques comprend :
- l'introduction de l'échantillon dans une colonne comprenant au moins deux phases stationnaires, la première phase stationnaire étant en un matériau capable d'adsorber les composés apolaires acycliques et la deuxième phase stationnaire étant en un matériau capable d'adsorber les composés aromatiques et les composés polaires,
- l'élution par un éluant, et
- la collecte de la fraction éluée.

On peut prévoir une étape de préparation du mélange de composés organiques préalable à l'étape de séparation. Cette étape de préparation peut notamment consister en une étape de mise en solution de l'échantillon du mélange avec ou sans étape de filtration ultérieure. On peut également procéder à un chauffage de l'échantillon. Ces étapes préparatoires peuvent également être combinées entre elles.

La première phase stationnaire est en un matériau capable d'adsorber les composés apolaires acycliques, notamment les n-alcanes. La deuxième phase stationnaire est en un matériau capable d'adsorber les composés aromatiques et les composés polaires (résines, asphaltènes).

La colonne de chromatographie peut en outre comprendre une troisième phase stationnaire, qui est optionnelle, en un matériau capable d'adsorber les composés dont la polarité est supérieure à la polarité des composés adsorbés par le matériau de la deuxième phase stationnaire, notamment les composés de masse moléculaire élevée et/ou les composés hétéroatomiques, notamment les hétéroalkyles comprenant au moins un atome choisi parmi : N, S et O, et/ou les résines et asphaltènes avec des masses moléculaires comprises entre 300 et 10 000 g/mol environ. L'homme du métier connait les matériaux susceptibles de satisfaire chacune de ces trois conditions et peut, par un simple test de routine, vérifier si un matériau convient ou non à cette utilisation. Il peut notamment tester, pour chaque famille de composés, si elle est retenue ou non sur un support, en procédant à un test d'élution sur le support testé à l'aide d'un solvant tel que de l'iso-octane ou de l'octane ou un mélange de ces solvants. Avantageusement, chacune des phases stationnaires est choisie pour retenir au moins 95 % en masse, par rapport à la masse totale de l'échantillon, des composés énumérés ci-dessus respectivement pour chaque phase stationnaire, mieux, au moins 98%, encore mieux, au moins 99%.

Avantageusement encore, les phases stationnaires sont choisies pour permettre l'élution au travers de la colonne de chromatographie d'au moins 70 % en masse, par rapport à la masse totale de l'échantillon, des composés iso-alcanes et cycloalcanes, et notamment les composés d'intérêt énumérés ci-dessus (notamment diamandoïdes), mieux au moins 75%, encore mieux, au moins 80%, préférentiellement au moins 85%, avantageusement au moins 90%, et encore plus avantageusement au moins 95%.

Avantageusement, la première phase stationnaire est en un matériau capable d'adsorber les n-alcanes, de préférence elle comprend une zéolithe de type aluminosilicate de sodium, avantageusement une zéolithe silicalite ZSM-5. Avantageusement, la première phase stationnaire est essentiellement constituée d'une zéolithe de type aluminosilicate de sodium, avantageusement une zéolithe silicalite ZSM-5.

Préférentiellement, la deuxième phase stationnaire est en un matériau capable d'adsorber les composés aromatiques et polaires, et de préférence elle comprend une silice imprégnée de nitrate d'argent. Préférentiellement, la deuxième phase stationnaire est essentiellement constituée d'une silice imprégnée de nitrate d'argent.

Avantageusement, la troisième phase stationnaire est en un matériau capable d'adsorber les composés de haut poids moléculaire et/ou hétéro-atomiques, notamment les hétéroalkyles comprenant au moins un atome choisi parmi : N, S et O et/ou les résines et asphaltènes avec des masses moléculaires comprises entre 300 et 10 000 g/mol environ. Préférentiellement, la troisième phase stationnaire comprend une silice choisie parmi une silice vierge, une silice greffée par des groupes aminoalkyles, notamment aminopropyles et une silice greffée par des groupes cyanoalkyles et préférentiellement par des groupes cyanopropyles. Préférentiellement, la troisième phase stationnaire est essentiellement constituée par une silice choisie parmi une silice vierge, une silice greffée par des groupes aminoalkyles, notamment aminopropyles et une silice greffée par des groupes cyanoalkyles et préférentiellement par des groupes cyanopropyles.

Le procédé de séparation d'iso-alcanes et de cycloalcanes à partir du mélange de composés organiques concerne plus particulièrement les mélanges de composés organiques provenant d'un gisement de pétrole brut, d'huiles de schiste ou de coupes de pétrole. Ce procédé concerne plus particulièrement les mélanges de composés organiques comprenant, outre un ou plusieurs composés tels que décrits ci-dessus et que l'on cherche à éliminer, un ou plusieurs composés d'intérêt choisis notamment parmi : des cycloalcanes, et l'on peut citer les diamandoïdes, notamment les adamantanes, les diamantanes et les triamantanes, les terpanes tricycliques, les hopanes, les stéranes ; des iso-alcanes, et l'on peut mentionner le prystane, le phytane.

Les phases stationnaires sont désignées sous le nom de première, seconde et troisième phase stationnaire dans l'ensemble de la description en référence à leur ordre d'introduction dans la colonne de chromatographie. Lorsque la colonne de chromatographie est éluée, l'éluant traverse tout d'abord la troisième phase stationnaire (si présente), puis la seconde, puis la première phase stationnaire.

Selon une version particulièrement préférée de l'invention, la colonne 1 comprend les trois phases stationnaires 11, 12, 13 successives suivantes, énoncées du bas vers le haut de la colonne 1, dans le sens opposé au sens d'élution :
- la zéolithe ZSM-5 comme première phase stationnaire 11, cette zéolithe étant avantageusement activée par un traitement thermique préalable,
- une silice imprégnée de nitrate d'argent comme deuxième phase stationnaire 12, et
- une silice greffée par des groupes cyanopropyles comme troisième phase stationnaire 13.

On désigne respectivement par M_{P1}, M_{P2}, M_{P3} les masses de la première, la seconde et la troisième phase stationnaire. Ces masses vérifient avantageusement les relations suivantes.

De préférence
M_{P2} ≤ M_{P1} ≤ 3 M_{P2}
M_{P3} ≤ M_{P1} ≤ 5 M_{P3}
M_{P3} ≤ M_{P2} ≤ 3 Mp₃

Encore plus préférentiellement,
M_{P2} ≤ M_{P1} ≤ 2 M_{P2}
M_{P3} ≤ M_{P1} ≤ 4 M_{P3}
M_{P3} ≤ M_{P2} ≤ 2,5 M_{P3}

Dans l'exemple mis en œuvre ci-dessous, les quantités de chaque phase stationnaire sont les suivantes :
MP₁ = 3,9-4,0g
M_{P2} = 2,3-2,4g
MP₃ = 1,3-1,4g

De préférence, la première étape de séparation est une étape de séparation par chromatographie basse pression, dans laquelle la colonne est soumise à une pression inférieure à 25 bars, avantageusement comprise entre 3 et 10 bars et, de préférence comprise entre 7 et 9 bars.

De préférence, dans la première étape de séparation l'éluant est choisi parmi l'iso-octane, le n-octane et leurs mélanges, avantageusement l'iso-octane.

La collecte de la fraction d'iso-alcanes et de cycloalcanes éluée peut être suivie directement par son introduction dans la colonne de chromatographie pour la mise en œuvre de la deuxième étape de séparation chromatographique.

Selon une variante, on peut prévoir une étape intermédiaire de préparation de la fraction d'iso-alcanes et de cycloalcanes. Cette préparation intermédiaire peut par exemple comporter : une filtration et/ou une concentration par évaporation partielle du solvant, une dilution.

Selon l'invention, le procédé peut comporter d'autres étapes, telles que notamment un rinçage de l'une ou des deux colonnes au moyen d'un ou de plusieurs solvants, en particulier avant ou après la mise en œuvre du procédé.

L'invention concerne en outre un kit permettant l'isolement de diamandoïdes par chromatographie liquide comprenant :
- une première colonne comprenant au moins deux phases stationnaires, la première phase stationnaire étant en un matériau capable d'adsorber les composés apolaires acycliques et la deuxième phase stationnaire étant en un matériau capable d'adsorber les composés aromatiques et les composés polaires, et
- une deuxième colonne comprenant une phase stationnaire en un matériau capable de former des complexes d'inclusion avec les diamandoïdes.

De préférence la première colonne comprend en outre une troisième phase stationnaire en un matériau capable d'adsorber les composés dont la polarité est supérieure à la polarité des composés adsorbés par le matériau de la deuxième phase stationnaire.

Les matériaux favoris pour la réalisation du kit ainsi que leur disposition et leurs proportions préférées sont ceux mentionnés ci-dessus pour le procédé.

Le kit comprend avantageusement des moyens permettant de relier la première colonne à des moyens de mise sous pression et la seconde colonne au dispositif HPLC envisagé ci-dessus. Il comprend avantageusement des moyens permettant l'introduction des échantillons concernés dans chacune des colonnes. Il peut en outre comprendre des moyens de collecte d'échantillon, des moyens de détection et d'analyse.

L'invention a encore pour objet un dispositif permettant l'isolement de diamandoïdes par chromatographie liquide comprenant :
- une première colonne comprenant au moins deux phases stationnaires, la première phase stationnaire étant en un matériau capable d'adsorber les composés apolaires acycliques et la deuxième phase stationnaire étant en un matériau capable d'adsorber les composés aromatiques et les composés polaires, et
- une deuxième colonne comprenant une phase stationnaire en un matériau capable de former des complexes d'inclusion avec les diamandoïdes,
- des moyens d'introduction de l'échantillon dans la première colonne,
- des moyens d'introduction d'un premier éluant dans la première colonne,
- éventuellement des moyens de mise sous pression de la première colonne,
- éventuellement des moyens de détection et d'analyse des fractions éluées en sortie de première colonne,
- des moyens de collecte des fractions éluées en sortie de première colonne,
- des moyens d'introduction dans la deuxième colonne, des fractions éluées en sortie de première colonne,
- des moyens d'introduction dans la deuxième colonne, d'un deuxième éluant,
- des moyens de mise sous pression de la deuxième colonne,
- éventuellement des moyens de détection et d'analyse des fractions éluées en sortie de deuxième colonne, et
- des moyens de collecte des fractions éluées en sortie de deuxième colonne.
- Il peut comprendre en outre des réservoirs pour les solvants, pour les produits à traiter et pour les produits collectés.

Le dispositif est avantageusement conçu pour permettre une continuité entre les deux étapes de séparation, tout d'abord d'une fraction d'iso-alcanes et de cycloalcanes à partir d'un mélange organique, ensuite de diamandoïdes à partir de la fraction d'iso-alcanes et de cycloalcanes.

Notamment, il comporte avantageusement des moyens d'automatisation du procédé, en particulier des moyens permettant le contrôle de l'introduction des échantillons dans les colonnes, et/ou la récupération des fractions éluées en sortie de colonne, et/ou le contrôle des moyens d'analyse, et/ou la récupération et le stockage des données analytiques. De tels moyens (ordinateurs et logiciels) sont bien connus de l'homme du métier.

Parmi les moyens de détection et d'analyse des fractions éluées en sortie de première colonne, on peut mentionner par exemple un réfractomètre différentiel, un détecteur à UV, un détecteur par ionisation de flamme (FID) ou encore un spectromètre de masse. Si le moyen d'analyse choisi présente un caractère destructeur, on prévoit avantageusement une dérivation, au moyen d'un by-pass, en amont des moyens de collecte, afin de faire transiter une petite partie des fractions éluées recueillies à la base de la colonne pour analyse, l'autre partie des fractions éluées étant recueillie dans les moyens de collecte.

Parmi les moyens de détection et d'analyse des fractions éluées en sortie de deuxième colonne, on peut mentionner un détecteur par diffraction de lumière évaporative ou ELSD, un réfractomètre différentiel ou RD, un spectromètre de masse ou MS, un spectromètre UV, un spectromètre irMS.

Ces procédés de séparation selon l'invention peuvent être mis en œuvre à l'échelle analytique (quelques dizaines de µl d'échantillon) ou à l'échelle préparative (quelques centaines de µl d'échantillon). Les conditions de séparation à l'échelle analytique par chromatographie liquide peuvent être directement extrapolées par l'homme du métier à l'échelle préparative au moyen de calculs de routine.

L'invention concerne encore un procédé de séparation de diamandoïdes à partir d'un mélange de composés organiques qui est mis en œuvre au moyen du kit tel que décrit ci-dessus ou au moyen du dispositif tel que décrit ci-dessus.

### Description de modes préférés de réalisation de l'invention

D'autres caractéristiques et avantages de l'invention apparaîtront à la lecture de la description qui suit d'un mode de réalisation préféré de l'invention, donné à titre d'exemple et en référence aux figures annexées.

### Kit de séparation

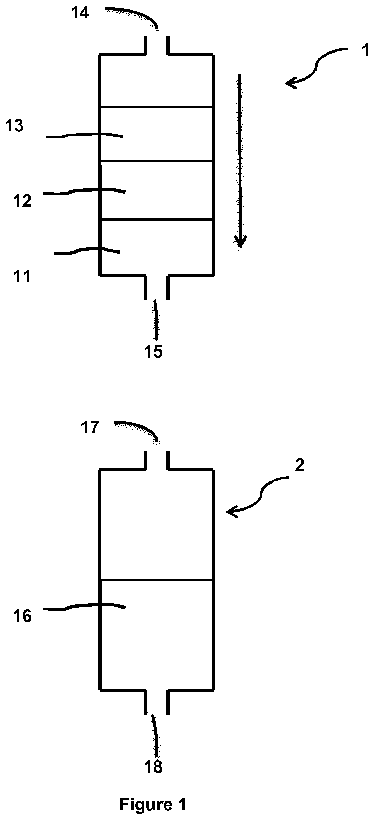

La figure 1 représente un schéma de principe d'un mode de réalisation du kit de séparation par chromatographie liquide selon l'invention.

Le kit illustré sur la figure 1 comprend une première colonne 1 et une deuxième colonne 2.

La première colonne 1 du kit est une colonne d'extraction sur phase solide (SPE). Elle comprend au moins deux phases stationnaires.

Dans le mode de réalisation représenté sur la figure 1, qui correspond à une version avantageuse du kit selon l'invention, la première colonne 1 comprend trois phases stationnaires 11, 12 et 13.

La première phase stationnaire 11 est en un matériau capable d'adsorber les composés apolaircs acycliques de l'échantillon et, plus particulièrement, les n-alcancs. Avantageusement, la première phase stationnaire 11 comprend une zéolithe choisie parmi les zéolithes de type aluminosilicate de sodium, en particulier celles appartenant à la famille des pentasiles. De préférence, dans ces zéolithes, le rapport molaire Si sur Al vérifie : Si/Al>10. On peut citer par exemple la zéolithe de type ZSM-5 de formule chimique :
NaₙAlₙSi₉₆₋ₙO₁₉₂·16H₂O (0<n<27). Ces zéolithes peuvent être utilisées directement ou être activées par un traitement thermique.

Préférentiellement, la première phase stationnaire comprend une zéolithe ZSM-5 commercialisée par la société Fisher Scientific. Avantageusement, avant sa mise en œ uvre, la zéolithe ZSM-5 est activée par chauffage, en particulier un chauffage à une température de l'ordre de 360°C pendant 12 heures est recommandé.

La deuxième phase stationnaire 12 est en un matériau capable d'adsorber les composés aromatiques et les composés polaires de l'échantillon (résines, asphaltènes). Avantageusement, la deuxième phase stationnaire 12 comprend une silice imprégnée de nitrate d'argent AgNO₃. On peut notamment mettre en œuvre la silice enregistrée sous le numéro CAS 7761-88-8 présentant une taille de particules de 62 µm et une densité de 4,35 g/ml à 25°C, cette silice étant notamment disponible auprès de la société Sigma Aldrich. Cette silice imprégnée de nitrate d'argent peut être utilisée directement, sans traitement préalable.

La troisième phase stationnaire 13 est en un matériau capable d'adsorber les composés de l'échantillon qui présentent une polarité supérieure à la polarité des composés adsorbés par le matériau de la deuxième phase stationnaire 12 et, plus particulièrement, les composés de haute masse moléculaire et/ou hétéro-atomiques, notamment les hétéroalkyles comprenant un ou plusieurs atomes choisis parmi : N, S et O. Avantageusement, la troisième phase stationnaire 13 comprend une silice qui peut être choisie parmi une silice vierge, une silice greffée par des groupes aminoalkyles, notamment aminoalkyles en C1-C5, de préférence aminopropyles et une silice greffée par des groupes cyanoalkyles, notamment cyanoalkyles en C1-C5, et, préférentiellement, par des groupes cyanopropyles. On peut en particulier utiliser une silice greffée par des groupes cyanopropyles présentant une taille de particules comprise entre 40 et 63 µm commercialisée par les sociétés Merck et VWR. Cette silice vierge ou greffée convenant pour la troisième phase stationnaire 13 peut être utilisée directement, sans traitement préalable.

Comme représenté sur la figure 1, la troisième phase stationnaire 13 est disposée au-dessus de la deuxième phase stationnaire 12, cette deuxième phase stationnaire 12 étant elle-même disposée au-dessus de la première phase stationnaire 11. La flèche représente le sens d'élution du solvant.

La deuxième colonne 2 du kit comprend une phase stationnaire en un matériau β-cyclodextrine capable d'inclure sélectivement les diamandoïdes. La deuxième colonne comprend une ouverture 17 permettant l'introduction de l'échantillon et une ouverture 18 permettant de récupérer la fraction éluée.

### Dispositif de séparation

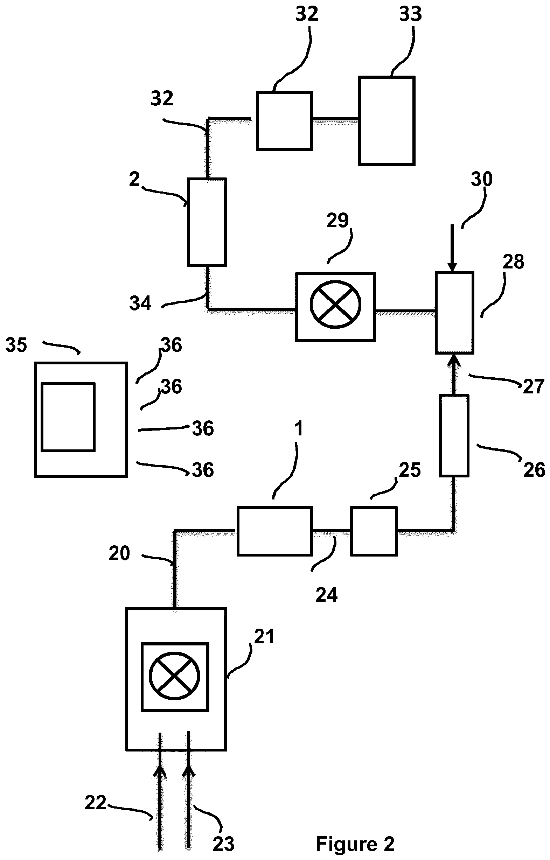

La figure 2 représente un schéma de principe d'un mode de réalisation du dispositif de séparation par chromatographie liquide et d'analyse selon l'invention, ce dispositif comprenant le kit de séparation par chromatographie selon l'invention tel que représenté sur la figure 1.

Le dispositif de séparation comprend la première colonne 1 qui est reliée, en amont, par des moyens de connexion 20 à un système de pompe 21. Ce système de pompe 21 est lui-même relié en amont à des moyens d'introduction 22 d'un premier éluant (ou phase mobile).

Les moyens d'introduction 23 de l'échantillon du mélange de composés organiques peuvent être connectés en entrée 14 de la colonne 1 au moyen d'un dispositif d'injection situé en aval du système de pompe 21 (éléments non représentés sur la figure 1). Mais, de manière avantageuse, et comme schématisé sur la figure 2, les moyens d'introduction 23 de l'échantillon sont également directement connectés au système de pompe 21, ce qui présente l'avantage de limiter les risques de pollution et/ou de perte de composés présents dans l'échantillon du fait de l'absence de joint.

La première colonne 1 est reliée, au niveau de sa sortie 15, par des moyens de connection 24, à des moyens de détection et d'analyse 25 des composés formant les fractions éluées. Ces moyens de détection et d'analyse 25, qui peuvent être constitués par un réfractomètre différentiel, sont eux-mêmes connectés à des moyens de collecte 26 des fractions éluées isolées à partir de l'échantillon.

On peut, selon une variante, prévoir que le dispositif de séparation selon l'invention comporte des moyens de collecte 26, mais pas de moyens d'analyse et que l'analyse des fractions éluées soit réalisée séparément.

Les moyens de collecte 26 sont connectés, par des moyens d'introduction 27, à un dégazeur 28, lui-même connecté à des moyens de mise sous pression, tels qu'une pompe haute pression 29. La pompe 29 est reliée à la deuxième colonne 2.

La colonne 2 est préférentiellement une colonne de chromatographie liquide à haute performance (HPLC).

Comme représenté sur la figure 2, les moyens d'introduction 30 du deuxième éluant sont connectés au dégazcur 28. Rien n'interdit toutefois que cette connexion soit établie directement au niveau de la pompe haute pression 29.

La deuxième colonne 2 est reliée, au niveau de sa sortie, par des moyens de connections 31, à des moyens de détection et d'analyse 32 des composés formant les fractions éluées. Ces moyens de détection et d'analyse 32 sont eux-mêmes connectés à des moyens de collecte 33 des fractions éluées isolées à partir de l'échantillon.

Ces moyens de détection et d'analyse 32 peuvent être constitués par un réfractomètre différentiel (RD) ou par un détecteur à diffusion de lumière (ELSD).

### Procédé de séparation

Le procédé de séparation selon l'invention peut être mis en œuvre en utilisant le dispositif de séparation et le kit décrits ci-dessus, en particulier lorsque l'on cherche à isoler les diamandoïdes à partir d'un échantillon d'un mélange de composés organiques, tel qu'un échantillon d'huile (ou pétrole) brute.

### Première étape de séparation par chromatographie liquide

Avant d'introduire l'échantillon comprenant un mélange de composés organiques, la première colonne 1 est soumise à un rinçage avec du solvant. De préférence, le solvant utilisé est identique à celui qui sera utilisé pour l'étape d'élution. Avantageusement on utilise de l'iso-octane.

L'échantillon du mélange de composés organiques est introduit dans le système de pompe 21 par les moyens d'introduction 23, puis dans la première colonne 1 par les moyens de connexion 20.

L'échantillon qui est introduit dans la première colonne 1, via les moyens d'introduction 23, le système de pompe 21 et les moyens de connexion 20, peut être un échantillon du mélange de composés organiques, tel qu'un échantillon d'huile brute, c'est-à-dire un mélange qui n'a subi aucun traitement préalable.

L'échantillon est introduit dans la colonne et le premier éluant est introduit, par les moyens d'introduction 22, dans le système de pompe 21 puis, par les moyens de connexion 20, dans la première colonne 1. Avec l'élution, les composés aromatiques et polaires sont adsorbés par la deuxième phase stationnaire 12 de la colonne 1 tandis que les composés apolaires acycliques et, notamment les n-alcanes, sont adsorbés par la première phase stationnaire 11.

L'élution par le premier éluant est effectuée en mode isocratique. Le premier éluant ou phase mobile, dont la composition n'est pas modifiée au cours de l'étape de séparation par chromatographie, est de l'iso-octane (à 100%).

La première étape de séparation par chromatographie liquide est conduite en colonne ouverte à basse pression. A cet effet, le dispositif selon l'invention comprend avantageusement les moyens 21 permettant la mise sous pression de la première colonne 1.

De préférence, cette étape de séparation est une étape de séparation par chromatographie dite "flash" ou basse pression. Typiquement, on applique, au moyen du système de pompe 21, une pression d'un gaz inerte pour la circulation de l'éluant le long de la première colonne 1. Cette pression appliquée, contrôlée au moyen d'un manomètre (non représenté) intégré au système de pompe 21, peut notamment être comprise entre 7 et 9 bars. Ainsi, l'étape de séparation est réalisée à des débits de phase mobile plus élevés qu'avec une séparation à pression atmosphérique.

Les fractions éluées recueillies à la base de la première colonne 1 sont ensuite acheminées, par les moyens de connexion 24, dans des moyens de détection et d'analyse 25 puis dans des moyens de collecte 26.

Les fractions éluées peuvent également être collectées directement en sortie de première colonne 1 et analysées ultérieurement. Notamment, la configuration des différents éléments du dispositif et les moyens employés pour l'analyse dépendent de l'objectif recherché, à savoir si l'on met en œuvre le procédé de séparation à l'échelle analytique ou à l'échelle préparative.

Ces moyens de détection et d'analyse 25 peuvent notamment être constitués par un réfractomètre différentiel (RD), un détecteur à UV (si on veut vérifier l'absence d'aromatiques dans les fractions collectée), un détecteur par ionisation de flamme (FID) ou encore un spectromètre de masse (MS).

Le procédé de séparation terminé, la première colonne 1 peut être détachée du dispositif et remplacée par une nouvelle colonne pour une séparation ultérieure.

### Etape, de préparation optionnelle

Les fractions éluées telles que celles collectées en sortie de première colonne 1 peuvent être soumises à une étape de préparation préalable avant la mise en œuvre de deuxième étape de séparation par chromatographie liquide.

Cette étape de préparation peut notamment consister en une étape de filtration et/ou de concentration, par exemple par évaporation partielle.

### Deuxième étape de séparation par chromatographie liquide

Les fractions éluées recueillies dans les moyens de collecte 26, qu'elles aient été, ou non, soumises à l'étape de préparation mentionnées ci-dessus, sont ensuite introduites, par les moyens d'introduction 27, dans le dégazeur 28 puis dans la pompe haute pression 29 et enfin dans la deuxième colonne 2.

Le deuxième éluant, de préférence un mélange pentane/isopropanol 98/2 vol/vol, est introduit, par les moyens d'introduction 30, dans le dégazeur 28, puis dans le système de pompe 29 et enfin dans la deuxième colonne 2 au travers de la connexion 34. Cette élution permet la complexation, dans la phase stationnaire de β-cyclodextrine, des diamandoïdes présents dans les fractions éluées et leur élution tardive par rapport aux autres composants du mélange d'iso-alcanes et de cycloalcanes.

L'élution par le deuxième éluant est effectuée en mode isocratique.

Cette deuxième étape de séparation par chromatographie liquide est, de préférence, mise en œuvre sous pression, soit au moyen d'un dispositif de chromatographie liquide à haute performance (HPLC), cette utilisation de l'HPLC étant la voie particulièrement préférée pour la mise en œuvre de cette deuxième étape de séparation. A cet effet, le dispositif selon l'invention comprend avantageusement des moyens permettant la mise sous pression de la deuxième colonne 2, grâce au système de pompe 29. Typiquement, on applique une pression qui peut notamment être comprise entre 19 et 20 bars.

Les fractions éluées recueillies à la base de la deuxième colonne 2 sont ensuite acheminées, par les moyens de connexion 31, dans des moyens de détection et d'analyse 32 puis dans des moyens de collecte 33.

Comme lors de la première étape de séparation, les fractions éluées peuvent également être collectées directement en sortie de deuxième colonne 2 et analysées ultérieurement. Notamment, la configuration des différents éléments du dispositif et les moyens employés pour l'analyse dépendent de l'objectif recherché, à savoir si l'on met en œuvre le procédé de séparation à l'échelle analytique ou à l'échelle préparative.

Ces moyens de détection et d'analyse 32 sont un détecteur à diffusion de lumière (ELSD), ou un réfractomètre différentiel (RD) mais les autres moyens mentionnés ci-dessus, tels que un détecteur à UV, un détecteur par ionisation de flamme (FID) ou encore un spectromètre de masse peuvent également être utilisés.

Le dispositif comprend des moyens d'automatisation du procédé, un ordinateur 35 et des logiciels, en particulier des logiciels de traitement de données, cet ordinateur étant relié par des connexions 36 (partiellement représentées) aux autres éléments du dispositif. Ces moyens permettent le contrôle de l'introduction des échantillons dans les colonnes, et/ou la récupération des fractions éluées en sortie de colonne, et/ou le contrôle des moyens d'analyse, et/ou la récupération et le stockage des données analytiques.

Bien entendu, lorsque l'échantillon de départ n'est pas constitué par un mélange de composés organiques, mais est formé d'un mélange d'iso-alcanes et de cycloalcanes, le procédé de séparation selon l'invention n'est mis en œuvre qu'à partir de la deuxième étape de séparation, cette deuxième étape étant éventuellement précédée de l'étape de préparation.

### Exemple

### - Etape 1

- Matière première : mélange de composés organiques de différentes origines, provenant de gisements de pétrole brut, d'huiles de schiste, de coupes de pétrole.
- Première colonne : colonne de 7,5 cm de longueur et de 1,7 cm de diamètre comprenant les trois phases stationnaires suivantes dans les quantités indiquées :
- la zéolithe ZSM-5 comme première phase stationnaire 11, MP₁ = 3,9-4,0g,
- une silice imprégnée de nitrate d'argent (CAS 7761-88-8) comme deuxième phase stationnaire 12, MP₂ = 2,3-2,4g,
   et
- une silice greffée par des groupes cyanopropyles (fournie par Merck) comme troisième phase stationnaire 13, MP₃ = 1,3-1,4g.
- Quantité d'échantillon introduite dans la première colonne : 300 µL

D'une manière générale, la quantité injectée dépend des caractéristiques de l'échantillon d'huile (en terme de pourcentage de n-alcanes notamment). En pratique, les règles suivantes peuvent être adoptées :
1) Dans le cas d'une huile dont le % massique en n-alcane C₃₀₊ est ≥ 1,5 % (et C₃₅₊> 0,5 %) : quantité max. à injecter en chromatographie flash : < 100 mg
équivalent huile totale.
2) Dans le cas d'une huile dont le % massique en n-alcane C₃₀₊ est ≥ 0,5 % (et C₃₅+ > 0,1 %) : quantité max. à injecter en chromatographie flash : ∼ 150-160 mg eq. HT.

Ces huiles sont généralement diluées en amont dans de l'iso-octane (facteur de dilution autour de 13, par exemple).
3) Sinon, injection de 250-260 mg eq. HT (soit un volume autour de 300 µL d'huile brute, non diluée).

Le procédé de séparation de la première étape a été conduit par chromatographie dite "flash", dans les conditions opératoires particulières indiquées dans le tableau 2 ci-dessous.

| | |
|---|---|
| Phase mobile | Iso-octane (100%) |
| Mode d'élution | isocratique |
| Débit | 1 ou 2 ml/min |
| Température | température ambiante (22°C) |
| Pression | 7 à 9 bars |
| Quantité injectée | Entre 100 et 250 mg équivalent huile totale |
| Durée de l'analyse | 10 min |

### - Etape 2

- β-cyclodextrine, colonne commerciale HPLC Cyclobond I 2000 - 5µm - dimensions : 250 mm x 4,6 mm ; fournisseur : Interchim (référence : 20024AST)-
- Quantité d'échantillon introduite dans la deuxième colonne : on introduit 10 µL de la fraction iso-alcanes et cyclo alcanes récupérée en sortie de première colonne
- Dispositif HPLC 1260 (Marque, Agilent).

Le procédé de séparation de la seconde étape a été conduit par chromatographie liquide à haute performance (HPLC), dans les conditions opératoires particulières indiquées dans le tableau 3 ci-dessous à l'aide du dispositif décrit ci-dessus.

**Tableau 3**

| | |
|---|---|
| Phase mobile | Pentane / isopropanol (98% / 2%) |
| Mode d'élution | isocratique |
| Débit | 1 ml/min |
| Température | température ambiante (22°C) |
| Pression | 19-20 bars |
| Volume d'échantillon injecté | 10 µL - 30 µL |
| Durée de l'analyse | 10 min |

Il peut être envisagé de répéter l'injection de la fraction d'iso-alcanes et de cycloalcanes. Les fractions collectées successivement peuvent alors réunies, puis concentrées par évaporation partielle.

Les fractions obtenues sont analysées. Les résultats sont reportés sur la figure 3 :
La courbe 1 représente l'analyse en réfractométrie différentielle d'une solution d'adamantane dans de l'iso-octane.
La courbe 2 représente l'analyse en ELSD (détection par diffraction de lumière évaporative) d'un mélange de 8 standards dans de l'iso-octane (adamantane ; diamantane ; ααα 20R Cholestane ; βαα 20R Cholestane ; 17β(H), 21 β(H) Hopane ; Phytane (2,6,10,14-tetraméthylhexadécane) ; Pristane (2,6,10,14-tetraméthylpentadécane) ; 3 méthyl pentadécane).

On repère les pics suivants :
a : pic d'iso-octane.
b : pic d'adamantane
c: pic d'iso-octane et des 6 standards d'isocyclo-alcanes (excepté adamantane et diamantane).
d: pic de diamantane.

L'histogramme reporté à la figure 4 montre par ailleurs que la fraction de diamandoïdes recueillie comprend des diamandoïdes dans des proportions à peu près constantes et supérieures à 80%, voire supérieures à 90% pour la plupart de ces composés, certains étant même proches des 100%. Il est précisé que les colonnes qui dépassent les 100% sont dues aux incertitudes analytiques.

On observe que le procédé de séparation selon l'invention, outre le fait d'être simple de mise en œuvre, sélectif en termes de collecte de diamandoïdes à partir d'une fraction d'iso-cycloalcanes et permettant cette collecte avec un bon niveau de rendement, est en outre rapide. A titre illustratif, on peut mentionner que le procédé de séparation ci-dessus a duré 10 minutes alors que le procédé de l'art antérieur nécessitait plus de 8 heures.

## Revendications

1. Procédé de séparation de diamandoïdes à partir d'un échantillon d'iso-alcanes et de cycloalcanes comprenant une étape de séparation par chromatographie liquide, cette étape de séparation comprenant :
- l'introduction de l'échantillon dans une colonne comprenant une phase stationnaire comprenant un matériau capable de former des complexes d'inclusion avec les diamandoïdes, le dit matériau étant la β-cyclodextrine,
- l'élution par un éluant choisi parmi les mélanges de n-alcane en C3-C8 et d'alcanol en C1-C4 avec un ratio en volume alcane/alcanol allant de 90/10 à 99,5/0,5 et
- la collecte des fractions éluées.

2. Procédé selon la revendication 1, dans lequel l'éluant est un mélange pentane / isopropanol (98% / 2% vol/vol).

3. Procédé selon l'une quelconque des revendications 1 à 2, dans lequel la colonne est une colonne HPLC reliée à un dispositif HPLC, le procédé étant mis en œuvre avec une pression dans la colonne allant de 10 à 30 bars, avantageusement de 15 à 25 bars.

4. Procédé d'isolement de diamandoïdes à partir d'un mélange de composés organiques par un procédé comprenant les étapes suivantes :
- une première étape de séparation par chromatographie liquide d'une fraction d'iso-alcanes et de cycloalcanes à partir du mélange de composés organiques,
- éventuellement une étape de préparation de la fraction d'iso-alcanes et de cycloalcanes, et
- une deuxième étape de séparation de diamandoïdes à partir d'un échantillon d'iso-alcanes et de cycloalcanes suivant le procédé selon l'une quelconque des revendications 1 à 3.

5. Procédé selon la revendication 4, dans lequel la première étape de séparation d'iso-alcanes et de cycloalcanes à partir du mélange de composés organiques comprend :
- l'introduction du mélange de composés organiques dans une colonne comprenant au moins deux phases stationnaires, la première phase stationnaire étant en un matériau capable d'adsorber les n-alcanes et
la deuxième phase stationnaire étant en un matériau capable d'adsorber les composés aromatiques et les composés polaires et ladite deuxième phase stationnaire comprend une silice imprégnée de nitrate d'argent,
- l'élution par un éluant, et
- la collecte de la ou des fractions d'iso-alcanes et de cycloalcanes éluées.

6. Procédé selon la revendication 5, dans lequel la première phase stationnaire comprend une zéolithe.

7. Procédé selon la revendication 5, dans lequel la colonne comprend en outre une troisième phase stationnaire en un matériau capable d'adsorber les composés dont la polarité est supérieure à la polarité des composés adsorbés par le matériau de la deuxième phase stationnaire, et, avantageusement, comprend une silice choisie parmi une silice vierge, une silice greffée par des groupes aminoalkyles, notamment aminopropyles et une silice greffée par des groupes cyanoalkyles et préférentiellement par des groupes cyanopropyles.

8. Procédé selon l'une quelconque des revendications 5 à 7, dans lequel la première étape de séparation est une étape de séparation par chromatographie basse pression, dans laquelle la phase liquide est éluée sous une pression inférieure à 25 bars, avantageusement comprise entre 3 et 10 bars et, de préférence, comprise entre 7 et 9 bars.

9. Procédé selon l'une quelconque des revendications 6 à 8, dans lequel l'éluant est choisi parmi l'iso-octane, le n-octane et leurs mélanges, avantageusement l'iso-octane.

10. Procédé selon l'une quelconque des revendications 5 à 9, qui comporte une étape intermédiaire de préparation de la fraction d'iso-alcanes et de cycloalcanes, cette préparation comportant une filtration et/ou une concentration par évaporation partielle du solvant.

11. Kit utilisable pour la mise en œuvre du procédé selon l'une quelconque des revendications 1 à 10, comprenant :
- une première colonne comprenant au moins deux phases stationnaires, la première phase stationnaire étant en un matériau capable d'adsorber les n-alcanes et
la deuxième phase stationnaire étant en un matériau capable d'adsorber les composés aromatiques et les composés polaires et ladite deuxième phase stationnaire comprend une silice imprégnée de nitrate d'argent,
éventuellement une troisième phase stationnaire en un matériau capable d'adsorber les composés dont la polarité est supérieure à la polarité des composés adsorbés par le matériau de la deuxième phase stationnaire, et
- une deuxième colonne comprenant une phase stationnaire en un matériau capable de former des complexes d'inclusion avec les diamandoïdes, ledit matériau étant la β-cyclodextrine.

12. Kit selon la revendication 11, qui comprend un ou plusieurs moyens choisis parmi :
des moyens permettant de relier la première colonne à des moyens de mise sous pression
des moyens permettant de relier la seconde colonne à un dispositif HPLC
des moyens permettant l'introduction des échantillons dans chacune des colonnes,
des moyens de collecte d'échantillon,
des moyens d'analyse.

13. Dispositif utilisable pour la mise en œuvre du procédé selon l'une quelconque des revendications 1 à 10, comprenant :
une première colonne comprenant au moins deux phases stationnaires, la première phase stationnaire étant en un matériau capable d'adsorber les n-alcanes et
la deuxième phase stationnaire étant en un matériau capable d'adsorber les composés aromatiques et les composés polaires et ladite deuxième phase stationnaire comprend une silice imprégnée de nitrate d'argent,
éventuellement une troisième phase stationnaire en un matériau capable d'adsorber les composés dont la polarité est supérieure à la polarité des composés adsorbés par le matériau de la deuxième phase stationnaire, et
une deuxième colonne comprenant une phase stationnaire en un matériau capable de former des complexes d'inclusion avec les diamandoïdes, ledit matériau étant la β-cyclodextrine,
des moyens d'introduction de l'échantillon dans la première colonne, des moyens d'introduction d'un premier éluant dans la première colonne éventuellement des moyens de mise sous pression de la première colonne éventuellement des moyens de détection et d'analyse des fractions éluées en sortie de première colonne
des moyens de collecte des fractions éluées en sortie de première colonne des moyens d'introduction dans la deuxième colonne, des fractions éluées en sortie de première colonne,
des moyens d'introduction dans la deuxième colonne d'un deuxième éluant,
des moyens de mise sous pression de la deuxième colonne éventuellement des moyens de détection et d'analyse des fractions éluées en sortie de deuxième colonne et
des moyens de collecte des fractions éluées en sortie de deuxième colonne. éventuellement des moyens de contrôle et d'automatisation du procédé, en particulier des moyens permettant le contrôle de l'introduction des échantillons dans les colonnes, et/ou la récupération des fractions éluées en sortie de colonne, et/ou le contrôle des moyens d'analyse, et/ou la récupération et le stockage des données analytiques,

14. Utilisation d'un procédé selon l'une quelconque des revendications 1 à 10 ou d'un kit selon l'une quelconque des revendications 11 à 12 ou d'un dispositif selon la revendication 13 pour isoler des diamandoïdes à partir d'un mélange de composés organiques, en particulier de pétrole brut ou d'une huile ou d'un mélange huileux issu du traitement du pétrole, notamment par raffinage.

## Patentansprüche

1. Verfahren zur Trennung von Diamantoiden aus einer Probe von Isoalkanen und Zykloalkanen, umfassend einen Trennungsschritt durch Flüssigchromatografie, wobei dieser Trennungsschritt umfasst:
- das Einleiten der Probe in eine Säule, umfassend eine stationäre Phase, umfassend ein Material, das geeignet ist, Inklusionskomplexe mit den Diamantoiden zu bilden, wobei das Material β-Zyklodextrin ist,
- die Elution durch ein Elutionsmittel, das ausgewählt ist unter den Gemischen von C3-C8-n-Alkan und C1-C4-Alkanol mit einem Volumenverhältnis Alkan/Alkanol von 90/10 bis 99,5/0,5 und
- das Sammeln der eluierten Fraktionen.

2. Verfahren nach Anspruch 1, bei dem das Elutionsmittel ein Pentan/Isopropanol-Gemisch (98%/2% Vol/Vol) ist.

3. Verfahren nach einem der Ansprüche 1 bis 2, bei dem die Säule eine HPLC-Säule ist, die mit einer HPLC-Vorrichtung verbunden ist, wobei das Verfahren mit einem Druck in der Säule von 10 bis 30 bar, vorteilhafterweise von 15 bis 25 bar, eingesetzt wird.

4. Verfahren zur Isolierung von Diamantoiden aus einem Gemisch von organischen Verbindungen durch ein Verfahren, umfassend die folgenden Schritte:
- ersten Trennungsschritt durch Flüssigchromatografie einer Fraktion von Isoalkanen und Zykloalkanen aus dem Gemisch von organischen Verbindungen,
- eventuell einen Schritt der Vorbereitung der Fraktion von Isoalkanen und Zykloalkanen, und
- einen zweiten Schritt der Trennung von Diamantoiden aus einer Probe von Isoalkanen und Zykloalkanen nach dem Verfahren nach einem der Ansprüche 1 bis 3.

5. Verfahren nach Anspruch 4, bei dem der erste Schritt der Trennung von Isoalkanen und Zykloalkanen aus dem Gemisch von organischen Verbindungen umfasst:
- das Einleiten des Gemisches von organischen Verbindungen in eine Säule, umfassend mindestens zwei stationäre Phasen, wobei die erste stationäre Phase aus einem Material ist, das geeignet ist, die n-Alkane zu adsorbieren, und wobei die zweite stationäre Phase aus einem Material ist, das geeignet ist, die aromatischen Verbindungen und die polaren Verbindungen zu adsorbieren, und wobei die zweite stationäre Phase ein mit Silbernitrat imprägniertes Siliziumoxid umfasst,
- die Elution durch ein Elutionsmittel, und
- das Sammeln der eluierten Fraktion(en) von Isoalkanen und Zykloalkanen.

6. Verfahren nach Anspruch 5, bei dem die erste stationäre Phase einen Zeolithen umfasst.

7. Verfahren nach Anspruch 5, bei dem die Säule ferner eine dritte stationäre Phase aus einem Material umfasst, das geeignet ist, die Verbindungen, deren Polarität größer als die Polarität der von dem Material der zweiten stationären Phase adsorbierten Verbindungen ist, zu adsorbieren, und vorteilhafterweise ein Siliziumoxid umfasst, das unter einem reinen Siliziumoxid, einem mit Aminoalkyl-, insbesondere Aminopropylgruppen gepfropften Siliziumoxid und einem mit Zyanalkyl- und vorzugsweise mit Zyanpropylgruppen gepfropften Siliziumoxid ausgewählt ist.

8. Verfahren nach einem der Ansprüche 5 bis 7, bei dem der erste Trennungsschritt ein Trennungsschritt durch Niederdruckchromatografie ist, bei dem die flüssige Phase unter einem Druck unter 25 bar, vorteilhafterweise zwischen 3 und 10 bar und vorzugsweise zwischen 7 und 9 bar, eluiert wird.

9. Verfahren nach einem der Ansprüche 6 bis 8, bei dem das Elutionsmittel unter Iso-Oktan, n-Oktan und ihren Gemischen, vorteilhafterweise dem Iso-Oktan, ausgewählt ist.

10. Verfahren nach einem der Ansprüche 5 bis 9, umfassend einen Zwischenschritt der Vorbereitung der Fraktion von Isoalkanen und Zykloalkanen, wobei diese Vorbereitung eine Filterung und/oder eine Konzentration durch teilweise Verdampfung des Lösungsmittels umfasst.

11. Set, das für den Einsatz des Verfahrens nach einem der Ansprüche 1 bis 10 verwendbar ist, umfassend:
- eine erste Säule, umfassend mindestens zwei stationäre Phasen, wobei die erste stationäre Phase aus einem Material ist, das geeignet ist, die n-Alkane zu adsorbieren, und
wobei die zweite stationäre Phase aus einem Material ist, das geeignet ist, die aromatischen Verbindungen und die polaren Verbindungen zu adsorbieren, wobei die zweite stationäre Phase ein mit Silbernitrat imprägniertes Siliziumoxid umfasst,
eventuell eine dritte stationäre Phase aus einem Material, das geeignet ist, die Verbindungen, deren Polarität größer als die Polarität der von dem Material der zweiten stationären Phase adsorbierten Verbindungen ist, zu adsorbieren, und
- eine zweite Säule, umfassend eine stationäre Phase aus einem Material, das geeignet ist, Inklusionskomplexe mit den Diamantoiden zu bilden, wobei das Material β-Zyklodextrine sind.

12. Set nach Anspruch 11, umfassend eines oder mehrere Mittel, die ausgewählt sind unter:
Mitteln, die es ermöglichen, die erste Säule mit Mitteln zum Druckaufbau zu verbinden,
Mitteln, die es ermöglichen die zweite Säule mit einer HPLC-Vorrichtung zu verbinden,
Mitteln, die das Einleiten der Proben in jede der Säulen ermöglichen,
Mitteln zum Sammeln von Proben,
Analysemitteln.

13. Vorrichtung, die für den Einsatz des Verfahrens nach einem der Ansprüche 1 bis 10 verwendbar ist, umfassend:
eine erste Säule, umfassend mindestens zwei stationäre Phasen, wobei die erste stationäre Phase aus einem Material ist, das geeignet ist, die n-Alkane zu adsorbieren, und
wobei die zweite Phase aus einem Material ist, das geeignet ist, die aromatischen Verbindungen und die polaren Verbindungen zu adsorbieren, wobei die zweite stationäre Phase ein mit Silbernitrat imprägniertes Siliziumoxid umfasst,
eventuell eine dritte stationäre Phase aus einem Material, das geeignet ist, die Verbindungen, deren Polarität größer als die Polarität der von dem Material der zweiten stationären Phase adsorbierten Verbindungen ist, zu adsorbieren, und
eine zweite Säule, umfassend eine stationäre Phase aus einem Material, das geeignet ist, Inklusionskomplexe mit den Diamantoiden zu bilden, wobei das Material β-Zyklodextrin ist,
Mittel zum Einleiten der Probe in die erste Säule,
Mittel zum Einleiten eines ersten Elutionsmittels in die erste Säule,
eventuell Mittel zum Druckaufbau in der ersten Säule,
eventuell Mittel zur Erfassung und Analyse der eluierten Fraktionen am Ausgang der ersten Säule,
Mittel zum Sammeln der eluierten Fraktionen am Ausgang der ersten Säule,
Mittel zum Einleiten der eluierten Fraktionen am Ausgang der ersten Säule in die zweite Säule,
Mittel zum Einleiten eines zweiten Elutionsmittels in die zweite Säule,
Mittel zum Druckaufbau in der zweiten Säule,
eventuell Mittel zur Erfassung und Analyse der eluierten Fraktionen am Ausgang der zweiten Säule, und
Mittel zum Sammeln der eluierten Fraktionen am Ausgang der zweiten Säule,
eventuell Mittel zur Steuerung und Automatisierung des Verfahrens, insbesondere Mittel, die die Steuerung des Einleitens der Proben in die Säulen und/oder die Wiedergewinnung der eluierten Fraktionen am Ausgang einer Säule und/oder die Steuerung der Analysemittel und/oder die Wiedergewinnung und Speicherung der analytischen Daten ermöglichen.

14. Verwendung eines Verfahrens nach einem der Ansprüche 1 bis 10 oder eines Sets nach einem der Ansprüche 11 bis 12 oder einer Vorrichtung nach Anspruch 13 zur Isolierung von Diamantoiden aus einem Gemisch von organischen Verbindungen, insbesondere Rohöl oder einem Öl oder einem Ölgemisch aus der Rohölverarbeitung, insbesondere durch Raffinieren.

## Claims

1. Method for separating diamandoids from a sample of iso-alkanes and cycloalkanes comprising a separation step by liquid chromatography, this separation step comprising:
- introducing the sample into a column comprising a stationary phase comprising a material capable of forming inclusion complexes with the diamandoids, said material being cyclodextrin,
- eluting with an eluent selected from mixtures of C3-C8 n-alkane and C1-C4 alkanol with an alkane/alkanol volume ratio ranging from 90/10 to 99.5/0.5, and
- collecting the eluted fractions.

2. Method according to claim 1, in which the eluent is a pentane/isopropanol mixture (98%/2% vol/vol).

3. Method according to any one of claims 1 to 2, in which the column is an HPLC column connected to an HPLC device, the method being implemented with a pressure in the column ranging from 10 to 30 bars, advantageously from 15 to 25 bars.

4. Method for isolating diamandoids from a mixture of organic compounds by a method comprising the following steps:
- a first separation step by liquid chromatography of a fraction of iso-alkanes and cycloalkanes from the mixture of organic compounds,
- optionally a step of preparation of the fraction of iso-alkanes and cycloalkanes, and
- a second separation step of the diamandoids from a sample of iso-alkanes and cycloalkanes according to the method according to any one of claims 1 to 3.

5. Method according to claim 4, in which the first separation step of iso-alkanes and cycloalkanes from the mixture of organic compounds comprises:
- introducing the mixture of organic compounds into a column comprising at least two stationary phases, the first stationary phase being of a material capable of adsorbing the n-alcanes and
the second stationary phase being of a material capable of adsorbing the aromatic compounds and the polar compounds, and said second stationary phase comprises a silver nitrate-impregnates silica, and
- eluting with an eluent, and
- collecting the fraction or fractions of eluted iso-alkanes and cycloalkanes.

6. Method according to claim 5, in which the first stationary phase comprises a zeolite.

7. Method according to claim 5, in which the column also comprises a third stationary phase of a material capable of adsorbing the compounds in which the polarity is greater than the polarity of the compounds adsorbed by the material of the second stationary phase and, advantageously, comprises a silica selected from a virgin silica, a silica grafted with aminoalkyl groups, in particular aminopropyl groups and a silica grafted with cyanoalkyl groups and preferentially with cyanopropyl groups.

8. Method according to any one of claims 5 to 7, in which the first separation step is a separation step by low pressure chromatography, in which the liquid phase is eluted under a pressure of less than 25 bars, advantageously comprised between 3 and 10 bars and, preferably, comprised between 7 and 9 bars.

9. Method according to any one of claims 6 to 8, in which the eluent is selected from iso-octane, n-octane and mixtures thereof, advantageously iso-octane.

10. Method according to any one of claims 5 to 9, which comprises an intermediate step of preparation of the fraction of iso-alkanes and cycloalkanes, this preparation comprising a filtration and/or a concentration by partial evaporation of the solvent.

11. Kit that can be used for the implementation of the method according to any one of claims 1 to 10, comprising:
- a first column comprising at least two stationary phases, the first stationary phase being of a material capable of adsorbing the n-alkanes and
the second stationary phase being of a material capable of adsorbing the aromatic compounds and the polar compounds and said second stationary phase comprises a silver nitrate-impregnates silica,
- optionally a third stationary phase of a material capable of adsorbing the compounds in which the polarity is greater than the polarity of the compounds adsorbed by the material of the second stationary phase, and
- a second column comprising a stationary phase of a material capable of forming inclusion complexes with the diamandoids, the material being β-cyclodextrin.

12. Kit according to claim 11, which comprises one or more means selected from:
means making it possible to connect the first column to pressurizing means,
means making it possible to connect the second column to an HPLC device,
means allowing the introduction of the samples into each of the columns,
means for collecting samples,
means of analysis.

13. Device that can be used for the implementation of the method according to any one of claims 1 to 10, comprising:
a first column comprising at least two stationary phases, the first stationary phase being of a material capable of adsorbing the n-alkanes and
the second stationary phase being of a material capable of adsorbing the aromatic compounds and the polar compounds, and
said a second stationary phase comprises a silver nitrate-impregnates silica,
optionally a third stationary phase of a material capable of adsorbing the compounds in which the polarity is greater than the polarity of the compounds adsorbed by the material of the second stationary phase, and
a second column comprising a stationary phase of a material capable of forming inclusion complexes with the diamandoids, the material being β-cyclodextrin.
means for introducing the sample into the first column,
means for introducing a first eluent into the first column,
optionally means for pressurizing the first column,
optionally means for detecting and analyzing the eluted fractions leaving the first column,
means for collecting the eluted fractions leaving the first column,
means) for introducing the eluted fractions leaving the first column into the second column,
means for introducing a second eluent into the second column,
means for pressurizing the second column,
optionally means for detecting and analyzing the eluted fractions leaving the second column, and
means for collecting the eluted fractions leaving the second column.
optionally means for monitoring and automation of the method, in particular means allowing the monitoring of the introduction of the samples into the columns, and/or the recovery of the eluted fractions leaving the column, and/or the monitoring of the analysis means, and/or the retrieval and storage of the analytical data.

14. Use of a method according to any one of claims 1 to 10 or of a kit according to any one of claims 11 to 12 or of a device according to claim 13 for isolating the diamandoids from a mixture of organic compounds, in particular of crude petroleum or of an oil or of an oily mixture originating from the treatment of the petroleum, in particular by refining.
